# DEMANDE DE BREVET EUROPEEN

(11) **EP 2 014 311 A2**
(43) Date de publication de la demande: **14.01.2009**
(21) Numéro de dépôt: 08290498.8
(22) Date de dépôt: 29.05.2008
(51) Int. Cl.: A61L 2/03, A61L 2/18, C02F 1/467, E01H 12/00

(54) **Procédé de préparation d'une solution de nettoyage de sable par électrolyse d'eau de mer et installation pour la mise en oeuvre d'un tel procédé**

(30) Priorité: 29.05.2007 FR 0755302
(71) Demandeur: Lasvaladas, Jean-Jacques, 16232 Vyronas (GR)
(72) Inventeur: Lasvaladas, Jean-Jacques, 16232 Vyronas (GR)
(74) Mandataire: Bredema

(57) **Abrégé**

L'invention concerne un procédé de préparation d'une solution de nettoyage de sable par électrolyse d'eau de mer, le procédé comprenant les étapes consistant à :
- transporter l'eau de mer depuis un réservoir (3), en cours de remplissage selon un débit déterminé, vers une cellule d'électrolyse (6), à l'intérieur de laquelle l'eau de mer subit une électrolyse sous l'action d'un courant d'intensité déterminée,
- rediriger dans le réservoir (3) l'électrolysât d'eau de mer obtenu en sortie de la cellule d'électrolyse (6), l'électrolysât comprenant des agents actifs,
le procédé comprenant une étape de re-circulation de la solution eau de mer / électrolysât de mer contenue dans le réservoir (3) au travers de la cellule d'électrolyse (6) jusqu'à ce que la solution contenue dans le réservoir (3) présente une concentration déterminée d'agents actifs.

## Description

L'invention concerne le domaine de la décontamination de terrains de sable.

L'invention concerne plus particulièrement un procédé de préparation d'une solution de nettoyage pour sable par électrolyse d'eau de mer.

La solution ainsi préparée est destinée notamment, mais non exclusivement, au nettoyage des plages de sable.

Des études scientifiques et des analyses bactériologiques ont permis de montrer que les sables de plages sont généralement chargés en germes pathogènes tels que des streptocoques, staphylocoques, coliformes, etc. De tels germes sont à l'origine de mycoses, d'affections urinaires ou cutanées. La présence de ces germes est due à la promiscuité de personnes déjà contaminées, à la souillure du sable par les personnes ou les animaux présents sur la plage. Elle est également lié aux traitements d'égouts à proximité des plages, et éventuellement au rejet des effluents dans la mer. La prolifération de ces germes est d'autant plus importante que la fréquentation des plages est élevée.

De nombreuses solutions ont été développées pour tenter de désinfecter les sables contaminés (utilisation de solutions à base d'acide hypochloreux, ou de solutions constituées de sulfate d'ammonium quaternaire, utilisation de solutions bactéricides formée par électrolyse d'eau de mer à une concentration de 250 à 450 mg de chlore/l). Toutefois, de telles solutions se sont avérées inefficaces ou du moins insuffisante dans la mesure où on ne parvient pas nécessairement à une désinfection totale. En outre, l'utilisation de telles solutions ont souvent des répercussions sur les faune et flore environnantes.

Pour tenter de pallier certains de ces inconvénients, il a été proposé dans la demande de brevet européen EP 0219 440 un procédé de décontamination d'une plage de sable ou de galets par traitement de la surface du sable et des galets. Le traitement s'effectue par pulvérisation en surface d'un électrolysât d'eau de mer à des doses comprises entre 1 et 10m3 de solution active par hectare de surface contaminée à traiter, l'électrolysât ayant une concentration d'agent actif comprise entre 100 et 500 mg/l, exprimée en équivalent chlore. Plus particulièrement, l'eau de mer pompée est envoyée à débit constant dans la cellule d'électrolyse. A la sortie de celle-ci, l'eau de mer contenant les espèces actives est, ou bien directement utilisée pour la pulvérisation, ou bien envoyée dans un réservoir de stockage. Il est alors possible, de ce réservoir de stockage, de préparer des solutions à pulvériser aux concentrations désirées. Pour ce faire, le concentré provenant du réservoir peut être redilué avec de l'eau de mer, un bloc de commande contrôlant le taux de dilution.

Le procédé de décontamination décrit dans la demande de brevet susmentionnée présente cependant un certain nombre d'inconvénients.

En particulier, l'installation associée au procédé ne permet d'obtenir qu'une solution de concentration donnée. Pour avoir des solutions à des concentrations particulières, il est nécessaire de procéder à des opérations complémentaires lesquelles consistent à diluer la concentration de la solution. Il s'ensuit des temps de préparation des solutions de décontamination long et requiert des équipements complémentaires.

Il est en outre nécessaire d'effectuer des contrôles à des étapes intermédiaires de la préparation de la solution, et en particulier des contrôles permanents lors des opérations de dilution de l'eau de mer électrolysée.

Le temps nécessaire à l'électrolyse de l'eau de mer auquel se rajoute le temps nécessaire aux contrôles intermédiaires de la préparation conduit à des durées de préparation de solution particulièrement longues. Or, ces durées peuvent être pénalisantes dans l'application de la solution au nettoyage du sable de plage, le nettoyage des plages devant être effectué en temps limité, généralement durant la nuit.

L'invention vise notamment à pallier les inconvénients de l'art antérieur précédemment décrit en proposant un procédé de préparation d'une solution de nettoyage de sable de mise en oeuvre simple, permettant une constance dans les solutions préparées, et ne nécessitant pas de contrôle intermédiaire de la concentration de la solution.

A cet effet, et selon un premier aspect, l'invention concerne un procédé de préparation d'une solution de nettoyage de sable par électrolyse d'eau de mer, le procédé comprenant les étapes consistant à :
- transporter l'eau de mer depuis un réservoir, en cours de remplissage selon un débit déterminé, vers une cellule d'électrolyse, à l'intérieur de laquelle l'eau de mer subit une électrolyse sous l'action d'un courant d'intensité déterminée,
- rediriger dans le réservoir l'électrolysât d'eau de mer obtenu en sortie de la cellule d'électrolyse, l'électrolysât comprenant des agents actifs,
le procédé comprenant une étape de re-circulation de la solution eau de mer / électrolysât de mer contenue dans le réservoir au travers de la cellule d'électrolyse jusqu'à ce que la solution contenue dans le réservoir présente une concentration déterminée d'agents actifs.

Ainsi, la mise en oeuvre simultanée de l'opération de remplissage du réservoir en eau de mer et l'opération de re-circulation de l'électrolysât dans la cellule d'électrolyse, permet de réduire le temps de préparation de la solution bactéricide (solution d'agents actifs contenus en concentration déterminée).

L'opération de re-circulation de l'électrolysât dans la cellule d'électrolyse permet également un traitement de l'eau de mer en continu. Une telle continuité dans la préparation de la solution bactéricide permet de générer des économies complémentaires de temps.

Par ailleurs, l'opération de re-circulation de l'électrolysât dans la cellule d'électrolyse, combinée à une opération de réglage préalable de l'intensité du courant appliqué dans la cellule d'électrolyse en fonction du débit de remplissage du réservoir, permet l'obtention d'une solution présentant une concentration en agents actifs constante et fiable.

Avantageusement, le remplissage du réservoir est effectué sous fort débit, de l'ordre de 8m³/heure).

Avantageusement, la re-circulation de la solution eau de mer / électrolysât de mer au travers de la cellule d'électrolyse est poursuivie jusqu'à ce que la solution atteigne un niveau N déterminé de remplissage du réservoir, le niveau de remplissage étant fonction de la concentration d'agents actifs de la solution.

Avantageusement, la re-circulation de la solution eau de mer / électrolysât de mer au travers de la cellule d'électrolyse est arrêtée lorsque la solution contenue dans le réservoir atteint une concentration de Chlore actif compris entre 800 et 1000 mg/litre. Ces valeurs correspondent à des concentrations permettant une désinfection efficace du sable, tout en ne présentant de risque ni pour les personnes en contact avec la solution, ni pour l'environnement, les germes indigènes non pathogènes présents naturellement dans le sable étant résistants à ce bactéricide naturel.

Selon un mode de réalisation particulier, le procédé comporte une étape préalable d'ajustement de l'intensité du courant appliquée dans la cellule d'électrolyse en fonction du volume du réservoir et du débit de l'eau de mer remplissant le réservoir. Il est ainsi possible d'obtenir des solutions présentant une concentration en agents actifs souhaitée.

Il peut être également prévu que l'intensité du courant appliqué dans la cellule d'électrolyse soit ajustée de manière à ce que le niveau N de remplissage corresponde à un remplissage total du réservoir. Ainsi, tout en obtenant des solutions présentant une concentration souhaitée en agents actifs, le procédé permet d'optimiser le volume de stockage à disposition. Par ailleurs, le remplissage de la cuve constitue un indicateur simple pour informer que la solution contenue dans le réservoir a la concentration souhaitée en ce qui concerne les agents actifs, et en conséquence pour lancer l'ordre d'arrêt du fonctionnement de la cellule d'électrolyse

Avantageusement, le remplissage du réservoir est réalisé à débit constant. De même, la circulation au travers de la cellule d'électrolyse est avantageusement à débit constant.

Avantageusement, l'eau de mer, préalablement à son déversement dans le réservoir, est soumise à une ou plusieurs opérations de filtration de matières organiques susceptibles de réagir avec les agents actifs de la solution.

Avantageusement, l'eau de mer, destinée à être déversée dans le réservoir, est pompée depuis un puits de stockage. Selon une configuration particulière, le puits est pourvu d'éléments empêchant la remontée de sable lors du pompage de l'eau de mer dans le puits.

Selon un deuxième aspect, l'invention concerne un procédé de nettoyage d'un terrain de sable. Le procédé consiste à appliquer par pulvérisation ou épandage-enfouissage sur le terrain une solution de nettoyage préparée selon le procédé tel que défini précédemment.

Afin de réaliser un traitement en profondeur du terrain de sable, l'opération de pulvérisation de la solution de nettoyage est effectuée conjointement avec une opération de criblage du terrain de sable.

Selon un autre aspect, l'invention concerne une installation pour la mise en oeuvre du procédé de préparation d'une solution de nettoyage tel que présenté. L'installation comporte une cellule d'électrolyse reliée en entrée et en sortie à un réservoir d'eau de mer et/ou d'électrolysât d'eau de mer par des conduits d'écoulement indépendants. Avantageusement, le réservoir est une citerne installée sur un véhicule de transport.

Avantageusement, l'installation comprend au moins un puits de stockage d'eau de mer relié au réservoir par un conduit d'écoulement.

D'autres objets et avantages de l'invention apparaîtront au cours de la description qui suit, faite en référence aux dessins annexés, dans lesquels :
- la figure 1 est une représentation schématique d'une installation de nettoyage de plages de sable selon une première configuration de l'invention ; et
- la figure 2 est une représentation schématique d'une installation de décontamination de plages de sable selon une deuxième configuration de l'invention.

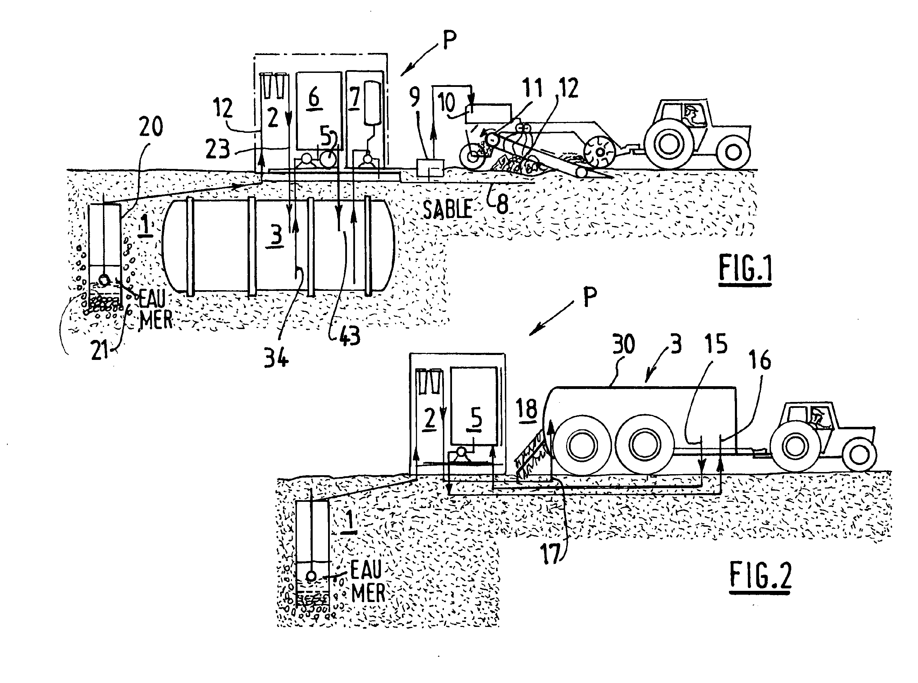

En relation avec la figure 1, il est décrit une installation de nettoyage P destinée à désinfecter un terrain ou sol sableux, tel qu'une plage de sable.

Dans le mode de réalisation décrit, l'installation de désinfection P comprend un module de préparation d'eau de mer en vue de sa transformation en solution de nettoyage et un module d'élaboration de ladite solution de nettoyage.

Concernant le module de préparation d'eau de mer, il comprend deux unités de filtrage 1, 2.

La première unité de filtrage 1 consiste en un puits 20, formé dans le sol, à proximité du rivage. Le puits 20 comporte une pluralité de filtres 21 destinés à limiter les remontées de sables. Les filtres utilisés sont avantageusement des filtres géo textiles. Par ailleurs, afin de faciliter leur nettoyage ou remplacement, les filtres sont avantageusement amovibles. Selon une configuration particulière, le puits 20 est un tube en polyéthylène haute densité.

La deuxième unité de filtrage 2 consiste en un poste de traitement pourvu d'un ou plusieurs filtres pour éliminer, de l'eau de mer, les matières organiques susceptibles de réagir avec les substances actives telles que le chlore dont sera pourvue l'eau de mer après son traitement aux fins de l'élaboration de la solution de nettoyage. Comme pour les filtres du puits 20, les filtres de la deuxième unité 2 sont des filtres amovibles. Ils peuvent donc être facilement nettoyés ou remplacés. La deuxième unité de filtrage 2 est située hors sol. Elle est raccordée en sortie de la première unité de filtrage 1 par l'intermédiaire d'un conduit d'écoulement 12.

Concernant le module de préparation de la solution de nettoyage, il comprend un réservoir ( cuve de stockage) 3, et une cellule d'électrolyse 6 monobloc.

Dans le mode de réalisation décrit, la cuve de stockage 3 est enterrée dans le sol. Toutefois, et comme on le verra plus loin dans la description en référence avec la figure 2, il peut être prévu une cuve de stockage hors sol, avantageusement mobile. Dans une telle configuration, la cuve de stockage sera avantageusement une citerne de grand volume (de l'ordre de 15 m³) installée sur un véhicule de transport approprié. En particulier, le véhicule sera approprié pour permettre le déplacement d'une lourde charge sur le sable sans endommager la surface de la plage. La cuve de stockage 3 est reliée en sortie de la deuxième unité de traitement 2 par un conduit d'écoulement 23.

La cellule d'électrolyse 6 est raccordée, en entrée et en sortie, à la cuve de stockage 3. Ainsi, l'eau de mer pompée depuis le puits 20, au travers des conduits d'écoulement 12 et 23, jusqu'à la cuve de stockage 3, est transportée par le conduit 34 vers la cellule d'électrolyse 6 au moyen d'une pompe 5. La cellule d'électrolyse est équipée d'un ensemble d'électrodes métalliques recouvertes d'une couche électrocatalytique. Les électrodes sont alimentées en courant continu. Les agents actifs produits par électrolyse sont essentiellement des ions hypochlorites, hypobromites, hypochloreux, et hypobromeux, lesquels ont, seuls ou en mélange, des pouvoirs désinfectants dont la durée est limitée dans le temps par l'action des ultraviolets.

L'eau de mer une fois électrolysée (électrolysât de mer) est renvoyée vers la cuve de stockage 3, par un conduit 43, indépendant du conduit 34.

Afin de limiter les distances entre chaque élément de l'installation, et donc ainsi réduire le temps et la puissance nécessaire pour transporter l'eau de mer/solution depuis la cuve de stockage 3 à la cellule d'électrolyse (et inversement), la cuve de stockage 3 est de préférence disposées dans le sol, sous la deuxième unité de traitement, ou du moins à proximité.

Le procédé d'élaboration de la solution bactéricide (électrolysât d'eau de mer) est le suivant :

L'eau de mer destinée à être traitée pour être utilisée comme solution bactéricide est pompée depuis le puits 20 où, débarrassée du sable et des graviers, elle est transportée vers la deuxième unité de filtrage 2 par l'intermédiaire du conduit d'écoulement 12. L'eau de mer traverse alors les filtres à cartouches disposés en entrée de l'unité de filtrage 2. Une fois filtrée, l'eau de mer est transportée dans la cuve de stockage 3. Une fois l'opération de remplissage de la cuve de stockage 3 démarrée, l'opération d'électrolyse peut commencer. Pour ce faire, l'eau de mer est pompée, depuis la cuve de stockage 3 vers la cellule d'électrolyse 6 par le biais du conduit d'écoulement 34. Une fois dans la cellule d'électrolyse 6, l'eau de mer subit une électrolyse pour se charger en agents actifs mentionnés plus haut. La solution d'électrolysât de mer obtenue (ou solution bactéricide) est alors retournée dans la cuve de stockage 3. En parallèle, l'opération de remplissage en eau de mer filtrée de la cuve de stockage 3 se poursuit. L'électrolysât d'eau de mer, de retour dans la cuve de stockage 3, se mélange donc avec l'eau de mer non électrolysée. Il est alors procédé à nouveau à une nouvelle circulation de cette solution entre la cellule d'électrolyse 6 et la cuve de stockage 3, cette circulation de la solution se poursuivant en continue jusqu'à ce qu'un niveau déterminé en terme de remplissage de la cuve et de concentration en agents actifs de la solution soit atteint. A cet instant, l'arrivée d'eau de mer dans la cuve de stockage 3 est stoppée et l'électrolyse arrêtée automatiquement.

Pour des questions de rentabilité, il sera avantageux de prévoir un niveau en terme de remplissage qui corresponde à un remplissage total de la cuve de stockage 3.

Le niveau déterminé en terme d'agents actifs correspond à une concentration en chlore actif comprise entre 800 et 1000 mg/l. Ces valeurs de concentration en chlore offrent une bonne désinfection du sable tout en étant sans risque pour les usagers et l'environnement. Pour obtenir une concentration comprise dans cet intervalle, l'intensité du courant produit par le redresseur de courant qui alimente l'électrolyseur est ajustée de sorte à mettre en route l'installation, et plus spécifiquement la mise en marche de la cellule électrolytique 4 en fonction du débit de remplissage de l'eau de mer et du volume de la cuve de stockage 3.

En d'autres termes, l'intensité sera ajustée de façon à obtenir une solution d'eau de mer ayant une valeur de concentration comprise dans cet intervalle lorsque la cuve de stockage est entièrement remplie.

La concentration en chlore actif obtenu dans ces conditions et par cet équipement est constante dans le temps. Il s'avère donc non nécessaire de contrôler, par analyse, la concentration en agents actifs à chaque cycle de production.

Une fois la cuve de stockage 3 pleine, la solution bactéricide peut être distribuée. Pour ce faire, une pompe de distribution 7 permet d'alimenter, à l'aide d'un réseau 8 enfoui dans le sable le long de la plage et de bornes de distribution 9, une cuve 10 tractée, soit par une machine de nettoyage mécanique 11 (cribleur), soit par une remorque équipée d'un dispositif de pulvérisation ou d'épandage-enfouissage. La solution bactéricide est pulvérisée ou épandue en même temps que le sable est brassé sur une profondeur de 5 à 15 centimètres.

La figure 2 décrit une autre configuration de l'installation de nettoyage selon l'invention.

Cette configuration prévoit une cuve de stockage 3 non enterrée dans le sol. Plus particulièrement, la cuve de stockage 3 consiste avantageusement en une citerne 30 de grand volume installée sur un véhicule de transport approprié pour assurer le déplacement d'une lourde charge sans endommager la surface de la plage. La citerne 30 est reliée en sortie de la deuxième unité de traitement 2 par un conduit d'écoulement 17, en sortie de la cellule d'électrolyse 6 par le conduit d'écoulement 16, et en entrée de la cellule d'électrolyse 6 par le conduit d'écoulement 15. Le procédé d'élaboration de la solution bactéricide est identique à celui décrit précédemment.

Aux fins de la distribution de la solution, une fois la citerne 30 de la remorque pleine, il suffira à l'opérateur de retirer les trois conduits 15, 16 et 17 reliant la citerne 30 à la cellule d'électrolyse 6 et à la deuxième unité de filtrage 2. La décontamination de la plage peut alors commencer par épandage-enfouissage 18 de l'électrolysât d'eau de mer en même temps que le brassage du sable sur une profondeur de 5 aà15 centimètres.

L'installation de décontamination de la figure 2 est particulièrement avantageuse dans le sens où l'opération de remplissage de la citerne 30 depuis une cuve de stockage contenant la solution bactéricide est supprimée.

La citerne 30 constitue en effet la cuve de stockage 3 vers laquelle l'eau de mer « partiellement » électrolysée ou l' « électrolysât final » d'eau de mer est directement dirigé.

Par eau de mer « partiellement » électrolysée, on entend l'eau de mer ayant déjà subie une ou plusieurs opération(s) d'électrolyse et devant subir au moins une autre opération d'électrolyse. Par électrolysât final, on entend l'électrolysât correspondant à la solution bactéricide comprenant la concentration souhaitée en agents actifs.

Le remplissage de la citerne 30 est donc réalisé au moment des opérations de transformation de l'eau de mer. Il n'y a donc plus de cuve de stockage d'électrolysât intermédiaire. Le remplissage de la citerne peut donc être effectué en dehors des heures de travail, laissant ainsi plus de temps pour procéder au nettoyage même des plages. Il découle que le gain de temps obtenu avec l'opération de remplissage se répercute sur la productivité des opérations de nettoyage. Il peut être en effet traité une surface de plage plus importante.

Le choix entre la configuration de l'installation illustrée sur la figure 1 et celle illustrée sur la figure 2 sera déterminé par les caractéristiques topologiques de la ou des plages à décontaminer et leurs dimensions d'une part, et à partir de critères économiques, tels que le coût de l'investissement, ou de fonctionnement lié au temps de traitement de la plage (le traitement étant en général effectué la nuit) d'autre part.

L'invention est décrite dans ce qui précède à titre d'exemple. Il est entendu que l'homme du métier est à même de réaliser différentes variantes de l'invention sans pour autant sortir de l'invention.

## Revendications

1. Procédé de préparation d'une solution de nettoyage de sable par électrolyse d'eau de mer, le procédé comprenant les étapes consistant à :
- transporter l'eau de mer depuis un réservoir (3), en cours de remplissage selon un débit déterminé, vers une cellule d'électrolyse (6), à l'intérieur de laquelle l'eau de mer subit une électrolyse sous l'action d'un courant d'intensité déterminée,
- rediriger dans le réservoir (3) l'électrolysât d'eau de mer obtenu en sortie de la cellule d'électrolyse (6), l'électrolysât comprenant des agents actifs,
le procédé comprenant une étape de re-circulation de la solution eau de mer / électrolysât de mer contenue dans le réservoir (3) au travers de la cellule d'électrolyse (6) jusqu'à ce que la solution contenue dans le réservoir (3) présente une concentration déterminée d'agents actifs.

2. Procédé de préparation d'une solution de nettoyage selon la revendication 1, **caractérisé en ce que** la re-circulation de la solution eau de mer / électrolysât de mer au travers de la cellule d'électrolyse est poursuivie jusqu'à ce que la solution atteigne un niveau N déterminé de remplissage du réservoir (3), le niveau de remplissage étant fonction de la concentration d'agents actifs de la solution.

3. Procédé de préparation d'une solution de nettoyage selon la revendication 1 ou la revendication 2, **caractérisé en ce que** la re-circulation de la solution eau de mer / électrolysât de mer au travers de la cellule d'électrolyse est arrêtée lorsque la solution contenue dans le réservoir (3) atteint une concentration de Chlore actif compris entre 800 et 1000 mg/litre.

4. Procédé de préparation d'une solution de nettoyage selon l'une quelconque des revendications précédentes, **caractérisé en ce qu'**il comporte une étape préalable d'ajustement de l'intensité du courant appliquée dans la cellule d'électrolyse (6) en fonction du volume du réservoir (3) et du débit de l'eau de mer remplissant le réservoir (3).

5. Procédé de préparation d'une solution de nettoyage selon l'une quelconque des revendications 2 à 4, **caractérisé en ce que** l'intensité du courant appliquée dans la cellule d'électrolyse est ajustée de manière à ce que le niveau N de remplissage corresponde à un remplissage sensiblement complet du réservoir (3).

6. Procédé de préparation d'une solution de nettoyage selon l'une quelconque des revendications précédentes, **caractérisé en ce que** le remplissage du réservoir (3) est réalisé à débit constant.

7. Procédé de préparation d'une solution de nettoyage selon l'une quelconque des revendications précédentes, **caractérisé en ce que** la circulation au travers de la cellule d'électrolyse (6) est à débit constant.

8. Procédé de préparation d'une solution de nettoyage selon l'une quelconque des revendications précédentes, **caractérisé en ce que** l'eau de mer, préalablement à son déversement dans le réservoir (3), est soumise à une ou plusieurs opérations de filtrage de matières organiques susceptibles de réagir avec les agents actifs de la solution.

9. Procédé de préparation d'une solution de nettoyage selon l'une quelconque des revendications précédentes, **caractérisé en ce que** l'eau de mer destinée à être déversée dans le réservoir (3) est pompée depuis un puit (20) de stockage.

10. Procédé de préparation d'une solution de nettoyage selon la revendication précédente, **caractérisé en ce que** le puits (20) est pourvu d'éléments empêchant la remontée de sable lors du pompage de l'eau de mer dans le puits (20).

11. Procédé de nettoyage d'un terrain de sable consistant à appliquer par pulvérisation ou épandage-enfouissage sur le terrain une solution de nettoyage préparée selon le procédé défini selon l'une quelconque des revendications précédentes.

12. Procédé de nettoyage d'un terrain de sable selon la revendication précédente, **caractérisé en ce que** la pulvérisation de la solution de nettoyage est réalisée conjointement avec une opération de criblage du terrain de sable.

13. Installation pour la mise en oeuvre du procédé de préparation d'une solution de nettoyage selon l'une quelconque des revendications 1 à 10, **caractérisée en ce qu'**elle comporte une cellule d'électrolyse reliée en entrée et en sortie à un réservoir (3) d'eau de mer et/ou d'électrolysât d'eau de mer par des conduits d'écoulement (34, 43) indépendants.

14. Installation pour la mise en oeuvre du procédé de préparation d'une solution de nettoyage selon la revendication 13, **caractérisée en ce que** le réservoir (3) est une citerne installée sur un véhicule de transport.

15. Installation pour la mise en oeuvre du procédé de préparation d'une solution de nettoyage selon la revendication 13 ou la revendication 14, **caractérisée en ce qu'**elle comprend au moins un puits de stockage (20) d'eau de mer relié au réservoir (3) par un conduit d'écoulement (12).
